# EUROPEAN PATENT APPLICATION

(11) **EP 3 984 350 A1**
(43) Date of publication of application: **20.04.2022**
(21) Application number: 20827401.9
(22) Date of filing: 29.05.2020
(51) Int. Cl.: A01G 7/00, A01M 1/00, G06Q 50/02

(54) **INFORMATION PROCESSING DEVICE AND METHOD**

(30) Priority: 17.06.2019 JP 2019111698
(71) Applicant: Bayer CropScience K.K., Tokyo 100-8268 (JP)
(72) Inventor: ITO, Satoshi, Tokyo 150-8360 (JP)
(74) Representative: BIP Patents
(86) International application number: PCT/JP2020/021366
(87) International publication number: WO 2020/255677

(57) **Abstract**

**Problem**

The present invention improves the accuracy of predicting the risk of diseases and pests.

**Solution**

An information processing device (40) comprising: an information acquisition unit (421) that acquires measurement information relating to the relative humidity inside a greenhouse; and a prediction unit (423) that generates, from the relative humidity measurement information, a feature amount indicating the dryness conditions inside the greenhouse, and predicts the risk of diseases and pests inside the greenhouse on the basis of the feature amount.

## Description

### Technical Field

The present invention relates to an information processing device and method.

### Background Art

Systems for protected horticulture in a plastic greenhouse, etc. have been developed in order to measure environmental conditions inside the plastic greenhouse by means of sensors, and to provide information relating to diseases and pests based on measurement results. For example, a system has been proposed for providing information by estimating locations where disease and pest damage will occur from measurement results of temperature and humidity inside a greenhouse (see Patent Document 1). Furthermore, a system has also been proposed for predicting a degree of infection by inputting measurement results of air temperature, rainfall and wind speed, etc. to an infection prediction model for diseases and pests (see Patent Document 2). By using this information, it is possible to take pre-emptive countermeasures, such as spraying agrochemicals.

### Prior Art Documents

### Patent Documents

Patent Document 1: JP 2015-119646 A
Patent Document 2: JP 2008-125496 A

### Summary of the Invention

### Problems to be Solved by the Invention

Condensation is known to increase the risk of diseases and pests in protected horticulture. In the case of gray mold, for example, if produce remains in a wet state for a long period of time because of condensation, there is likely to be an increased risk of infection due to fungal spores adhering to the produce. It is therefore possible to predict the risk of infection by determining a state of wetness in accordance with the temperature and humidity measured by means of sensors inside the plastic greenhouse.

Under dry conditions, on the other hand, spores may be dispersed after the infection, although this depends on the type of disease. For example, it is known that powdery mildew spores absorb moisture themselves, or the water content of conidiospores is especially high, so germination also occurs under dry conditions. It is thus difficult to predict a risk which increases under dry conditions by determining the state of wetness in the manner described above.

The purpose of the present invention lies in improving the accuracy of predicting the risk of diseases and pests.

### Means for Solving the Problems

One mode of the present invention provides an information processing device (40) comprising: an information acquisition unit (421) for acquiring measurement information of relative humidity inside a plastic greenhouse; and a prediction unit (423) for generating a feature value representing a dryness condition inside the plastic greenhouse from the measurement information of the relative humidity, and predicting a risk of diseases and pests inside the plastic greenhouse on the basis of the feature value.

Another mode of the present invention provides a method for predicting a risk of diseases and pests in a plastic greenhouse, the method comprising: a step of acquiring measurement information of relative humidity inside the plastic greenhouse; and a step of generating a feature value representing a dryness condition inside the plastic greenhouse from the measurement information of the relative humidity, and predicting a risk of diseases and pests inside the plastic greenhouse on the basis of the feature value.

### Advantage of the Invention

The present invention makes it possible to improve the accuracy of predicting a risk of diseases and pests.

### Brief Description of the Drawings

[Fig. 1] is a diagram showing a configuration of an information provision system comprising an information processing server according to the present mode of embodiment.
[Fig. 2] is a diagram showing a configuration of the information processing server.
[Fig. 3] is a flowchart showing a processing sequence by which the information processing server generates a prediction model.
[Fig. 4] is a graph showing a correlation of relative humidity and an increase rate of diseases and pests.
[Fig. 5] is a flowchart showing a processing sequence by which the information processing server predicts a risk of diseases and pests.
[Fig. 6] is a diagram showing an exemplary display of prediction information of the risk of diseases and pests.

### Mode for Implementing the Invention

A mode of embodiment of the information processing device and method according to the present invention will be described below with reference to the drawings. The configuration described below is an example (representative example) of one mode of embodiment of the present invention, and the present invention is not limited to the configuration described below.

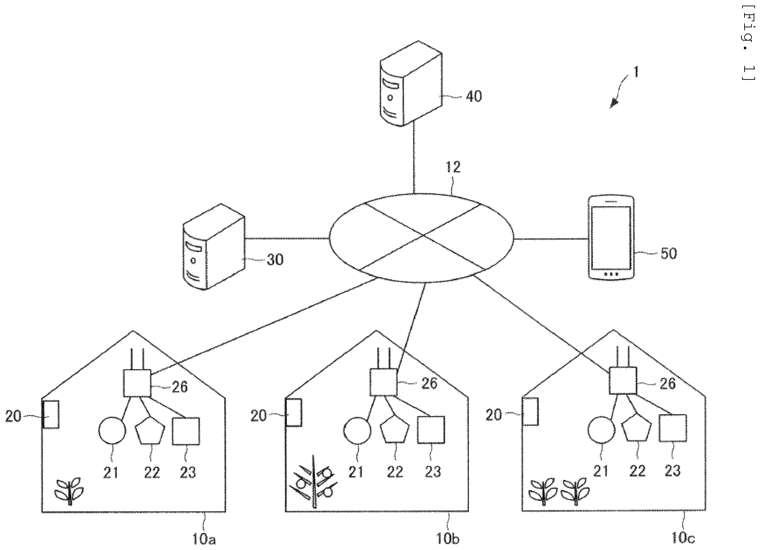

Fig. 1 shows an information provision system 1 according to a mode of embodiment of the present invention.

The information provision system 1 measures a temperature and a relative humidity, etc. inside and outside plastic greenhouses 10a-10c, and provides prediction information of a risk of diseases and pests in the plastic greenhouses 10a-10c in accordance with a measurement result. Fig. 1 shows an example in which prediction information for the three plastic greenhouses 10a-10c is provided, but there is no particular limitation as to the number of plastic greenhouses which may be provided with the prediction information, and the prediction information may be provided to one or more plastic greenhouses.

As shown in fig. 1, the information provision system 1 comprises: a plurality of sensors 21-23, a communication device 26, a weather server 30, an information processing server 40, and a user terminal 50. The communication device 26, weather server 30, information processing server 40 and user terminal 50 are communicably connected to one another via a network 12. The network 12 may comprise the Internet, a telephone network, or a LAN (local area network), etc.

The sensors 21-23 are provided inside the plastic greenhouses 10a-10c, and measure environmental conditions inside the plastic greenhouses 10a-10c at fixed intervals of 10 minutes or the like, for example. Examples of environmental conditions which may be cited include: temperature, relative humidity, solar irradiance, carbon dioxide concentration, wind speed, terrestrial heat, and soil moisture content, etc. In this mode of embodiment, the sensor 21 measures temperature, the sensor 22 measures relative humidity, and the sensor 23 measures solar irradiance, but sensors for measuring other environmental conditions such as carbon dioxide concentration may equally be provided.

The communication device 26 sends the temperature, relative humidity and solar irradiance, etc. measured by means of the respective sensors 21-23 to the information processing server 40 as measurement information of the environmental conditions inside the plastic greenhouses 10a-10c.

Furthermore, a control device 20 for adjusting the environmental conditions may be provided inside the plastic greenhouses 10a-10c. The communication device 26 may acquire required information from the control device 20 to generate operating information of the control device 20, and may send this operating information to the information processing server 40. An example of the control device 20 which may be cited is a device for controlling opening and closing of an air conductor, a sprinkler, a sun-shading curtain, or a window, etc.

Communication between the communication device 26 and the sensors 21-23 and control device 20 takes place by wireless communication such as BLE (Bluetooth (registered trademark) Low Energy), or Wi-Fi (registered trademark), but wired communication is equally possible.

The weather server 30 sends measurement information of weather conditions outside the plastic greenhouses 10a-10c to the information processing server 40. Examples of weather conditions which may be cited include air temperature, relative humidity, solar irradiance, rainfall, and wind speed, etc. in each region. The weather server 30 may send not only measurement information to the information processing server 40, but also prediction information of weather conditions such as a weather forecast.

The information processing server 40 is an information processing device which acquires from the communication device 26 the measurement information of the environmental conditions including the relative humidity inside the plastic greenhouses 10a-10c, and predicts the risk of diseases and pests in the plastic greenhouses 10a-10c on the basis of the measurement information acquired. The information processing server 40 is capable of generating and outputting prediction information of the risk of diseases and pests on the basis of a prediction result.

The user terminal 50 is a mobile telephone, a tablet, or a PC (personal computer), etc., for example. The user terminal 50 is used by a user such as a farmer managing the plastic greenhouses 10a-10c, and displays the prediction information sent from the information processing server 40.

### (Information processing server)

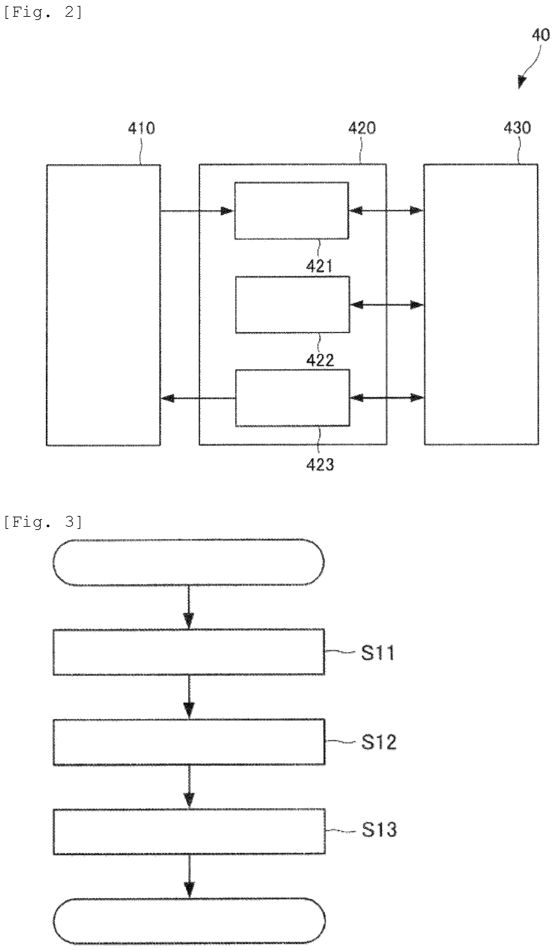

Fig. 2 shows an exemplary configuration of the information processing server 40.

As shown in fig. 2, the information processing server 40 comprises a communication unit 410, a control unit 420, and a memory unit 430.

The communication unit 410 is an interface for communicating with external devices on the network 12, such as the communication device 26, the weather server 30, and the user terminal 50.

The control unit 420 controls operation of the information processing server 40.

Furthermore, the control unit 420 predicts the risk of diseases and pests in each of the plastic greenhouses 10a-10c. For the purposes of this prediction, the control unit 420 comprises an information acquisition unit 421, a learning unit 422 and a prediction unit 423, as shown in fig. 2. The information acquisition unit 421, the learning unit 422 and the prediction unit 423 may be realized by means of software processing in which a processor such as a CPU (central processing unit) executes a program stored in the memory unit 430 or another recording medium such as a memory, or the above units may be realized by means of hardware such as an ASIC (application specific integrated circuit).

The information acquisition unit 421 acquires measurement information of the relative humidity inside the plastic greenhouses 10a-10c from the communication device 26 via the communication unit 410. The information acquisition unit 421 is capable of acquiring measurement information of environmental conditions inside the plastic greenhouses 10a-10c other than relative humidity, and operating information of the control device 20 from the communication device 26, and is capable of acquiring measurement information of the weather conditions from the weather server 30. The information acquisition unit 421 may acquire the measurement information at predetermined intervals, such as intervals of 10 minutes, for example. The information acquisition unit 421 saves the acquired information in the memory unit 430.

The learning unit 422 generates a feature value representing a dryness condition from the measurement information of the relative humidity saved in the memory unit 430, and generates a prediction model for the risk of diseases and pests by using this feature value. The prediction model generated is saved in the memory unit 430.

The prediction model may be a prediction formula capable of calculating the risk of diseases and pests using the feature value as a variable, or it may be a table in which predicted risks are pre-established in relation to variables. Furthermore, the prediction model may be a model which is generated by means of machine learning using the feature value representing the dryness condition as input data, and using the risk of diseases and pests under that dryness condition as teaching data. An example of a prediction model afforded by machine learning which has a higher level of prediction accuracy will be described in this mode of embodiment.

The prediction unit 423 generates a feature value representing the dryness condition inside the plastic greenhouses 10a-10c from the measurement information of the relative humidity inside the plastic greenhouses 10a-10c acquired by means of the information acquisition unit 421, and predicts the risk of diseases and pests inside the plastic greenhouses 10a-10c on the basis of the feature value generated. Specifically, the prediction unit 423 inputs the generated feature value to the prediction model generated by means of the learning unit 422 and can thereby acquire a prediction result of the risk of diseases and pests inside the plastic greenhouses 10a-10c.

The memory unit 430 stores various types of information acquired by means of the information acquisition unit 421. Furthermore, the memory unit 430 stores the prediction model generated by means of the learning unit 422. A large-capacity storage medium such as a hard disk may be used as the memory unit 430.

### (Processing by the information processing server)

When produce becomes infected by a fungus that causes disease, the fungus generally passes through an incubation period before an outbreak of disease. Likewise in the case of insect damage also, insect eggs adhere to (infect) the produce and the insects which have hatched after a given period (incubation period) cause feeding damage (outbreak of disease). Fungal spores and insect eggs causing a subsequent infection are formed by the outbreak of disease. In order to pre-empt the disease and pest damage arising from this cycle, measures such as spraying agrochemicals or blowing air are effectively taken before infection or before the outbreak of disease, and especially before infection.

Among diseases and pests, there are those for which an outbreak of disease occurs under dry conditions. For diseases and pests such as these, it is possible to predict the risk of an outbreak of disease or the risk of infection occurring after an outbreak of disease according to whether or not the interiors of the plastic greenhouses 10a-10c are under dry conditions. The information processing server 40 generates the prediction model of the risk of diseases and pests which increases under a specific dryness condition, by generating a feature value representing the dryness condition using past measurement information of the relative humidity.

Fig. 3 shows a processing sequence by which the information processing server 40 generates the prediction model.

As shown in fig. 3, in the information processing server 40, the information acquisition unit 421 acquires from the communication device 26 the measurement information of the relative humidity measured inside the plastic greenhouses 10a-10c by means of the sensor 22, and saves this measurement information in the memory unit 430 (step S11).

The learning unit 422 generates a feature value representing the dryness condition inside the plastic greenhouses 10a-10c from the measurement information of the relative humidity saved in the memory unit 430 (step S12) .

The following feature values (f11)-(f17) may be cited as examples of feature values generated from the measurement information of the relative humidity. Higher numerical values for the feature values (f11)-(f13) below mean that the interior of the plastic greenhouses 10a-10c is under a dryness condition with a higher risk of diseases and pests.
(f11) A mean value for one day of a risk (A) of diseases and pests when values of relative humidity acquired at predetermined intervals (e.g., 10 minutes) are input to a relational expression expressing a correlation of relative humidity and risk of diseases and pests;
(f12) a cumulative value for one day of the risk (A);
(f13) a mean value for one day or a cumulative value of values of 1, with respect to values obtained by binarizing to a value of 1 when the risk (A) is higher than a threshold, or to a value of 0 when the risk (A) is lower than the threshold;
(f14) a mean humidity for one day;
(f15) a mean value for one day or a cumulative value of values of 1, with respect to values obtained by binarizing to a value of 1 when the relative humidity is higher than a threshold, or to a value of 0 when the relative humidity is lower than the threshold;
(f16) a value obtained by subtracting the relative humidity from 100 (%); and
(f17) the reciprocal of the relative humidity.

Provided that the relational expression expressing a correlation of relative humidity and risk of diseases and pests is capable of expressing the risk in relation to relative humidity, then the relational expression may output an infection rate, a disease outbreak rate, or an increase rate, etc. of the disease or pest in relation to relative humidity. The dryness condition which increases the risk of diseases and pests varies according to the type of disease or pest, so a relational expression is prepared on the basis of the correlation between the two for each type of disease or pest.

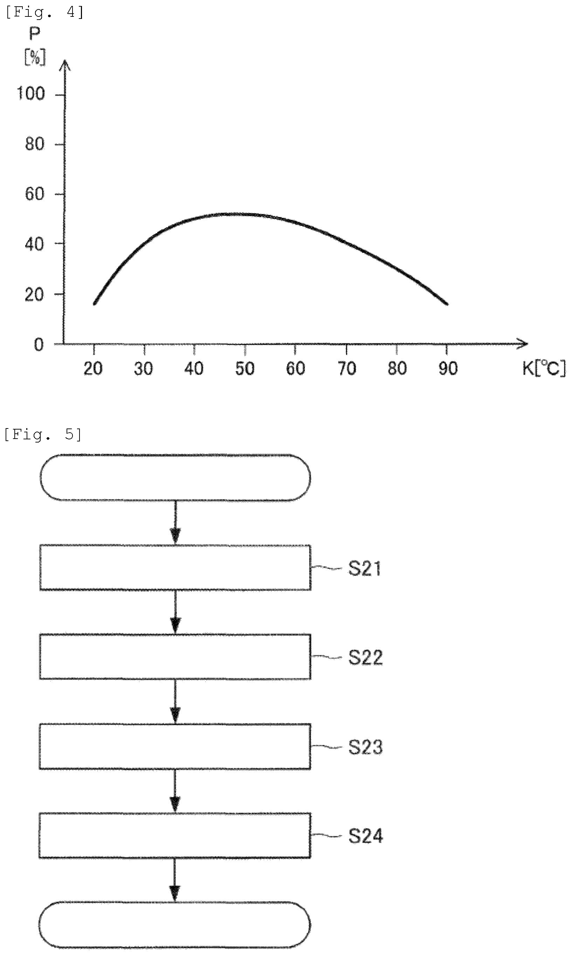

Fig. 4 shows an exemplary correlation of a relative humidity K (%) and an increase rate P (%) of a disease *a.*

As shown in fig. 4, the increase rate P of the disease a slowly rises as the relative humidity K increases, and the increase rate P is at a maximum in the region where the relative humidity K is 40-50%. When the relative humidity K exceeds 50%, the increase rate P slowly declines. That is to say, the risk of disease a increases under a dryness condition where the relative humidity is 40-50%.

The learning unit 422 may also generate a feature value representing a dryness condition from measurement information of environmental conditions other than relative humidity, such as temperature or solar irradiance inside the plastic greenhouses 10a-10c.

For example, the following feature values (f21)-(f26) may be generated from measurement information of the temperature measured by means of the sensor 21. Higher numerical values for the feature values (f21)-(f23) and (f26) below mean that the interior of the plastic greenhouses 10a-10c is under a dryness condition with a higher risk of disease. It should be noted that the following relational expression is prepared for each type of disease or pest on the basis of the correlation between temperature and risk of diseases and pests, in the same way as with relative humidity above.
(f21) A mean value for one day of a risk (B) of diseases and pests when values of temperature acquired at predetermined intervals (e.g., 10 minutes) are input to a relational expression expressing a correlation of temperature and risk of diseases and pests;
(f22) a cumulative value for one day of the risk (B); (f23) a mean value for one day or a cumulative value of values of 1, with respect to values obtained by binarizing to a value of 1 when the risk (B) is higher than a threshold, or to a value of 0 when the risk (B) is lower than the threshold;
(f24) a mean temperature for one day;
(f25) a mean value for one day or a cumulative value of values of 1, with respect to values obtained by binarizing to a value of 1 when the temperature is higher than a threshold, or to a value of 0 when the temperature is lower than the threshold; and
(f26) a cumulative value for one day of values (A*B) obtained by multiplying the risk (B) by the risk (A), which are each calculated at predetermined intervals.

Temperature differences have a considerable effect on the dryness condition, so the learning unit 422 may also generate the following feature values (f31) and (f32) from measurement information of the temperature, as feature values representing the dryness condition.
(f31) A temperature difference of a maximum temperature and a minimum temperature for one day; and
(f32) a difference in two different percentile values of temperature for one day (e.g., the difference between the 75^{th} percentile value and the 25^{th} percentile value) .

The learning unit 422 may use a feature value generated from measurement information of the humidity for a fixed period in the past as a primary feature value, and may further generate a secondary feature value representing a dryness condition inside the plastic greenhouses 10a-10c in the fixed period from this primary feature value. Examples of the secondary feature value which may be cited include a mean value or a percentile value, etc. of the primary feature value generated during a fixed period such as one week or one month.

By using the secondary feature value representing a longer-term dryness condition than the primary feature value, it is possible to predict the risk of diseases and pests on the basis of the length of a sustained period of a dry state, further improving the prediction accuracy. An outbreak of diseases or pests is likely to occur under a continued long-term dryness condition of one or two weeks, or one month, etc., rather than under a short-term dryness condition of the order of several hours. Accordingly, when the fixed period for generating the secondary feature value is a period in week units or month units, rather than hour units, a dryness condition under which the risk increases can be estimated more precisely, which is preferable.

When the learning unit 422 generates a feature value representing a dryness condition, it uses the feature value as input data, uses the risk of diseases and pests under that dryness condition as teaching data, and generates the prediction model of the risk of diseases and pests by means of machine learning (step S13). The learning unit 422 saves the generated prediction model in the memory unit 430.

Examples of machine learning for generating the prediction model that may be cited include: linear regression, a filter such as a Kalman filter, a support vector machine, a decision tree such as a random forest, a nearest neighbor method, a neural network such as deep learning, and a Bayesian network. One of the above types of machine learning may be used alone, or two or more may be combined for use.

The learning unit 422 may generate the prediction model by using, as input data, the feature value representing the dryness condition together with information affecting the dryness condition. By using multiple input data items, it is possible to make a comprehensive prediction, further improving the prediction accuracy.

For example, the weather conditions outside the plastic greenhouses 10a-10c affect the dryness condition inside the plastic greenhouses 10a-10c. The information acquisition unit 421 may therefore acquire measurement information of the weather conditions outside the plastic greenhouses 10a-10c from the weather server 30, and the learning unit 422 may use this measurement information of the weather conditions as one item of input data. It is thereby possible to make a prediction which takes account of the effect of weather conditions, further improving the prediction accuracy.

Furthermore, cultivation conditions inside the plastic greenhouses 10a-10c, for example cultivation conditions such as a sowing area or density, and a growth stage, also affect the dryness condition. The information acquisition unit 421 may therefore acquire cultivation information in the plastic greenhouses 10a-10c, and the learning unit 422 may use this cultivation information as one item of input data. It is thereby possible to make a prediction which takes account of the effect of produce cultivation conditions, further improving the prediction accuracy.

The cultivation information is information relating to cultivation conditions such as the type of produce, cultivation amount, growth condition and cultivation ground, for example. The type of produce is a category such as cucumber or tomato, for example. Examples of the cultivation amount that may be cited include the sowing area, number of plants, and planting density in the plastic greenhouses. The planting density may be calculated by dividing the number of plants by the sowing area. Examples of growth conditions that may be cited include the number of days elapsed from the planting date, and a growth stage estimated from the number of days since planting. The cultivation ground is a category such as soil culture or water culture, for example. This cultivation information is input from the user terminal 50, for example, and thereby saved in advance in the memory unit 430 of the information processing server 40. The information relating to the number of days after planting and the growth stage may be estimated by the information acquisition unit 421 in accordance with the time elapsed from the planting date. That is to say, the information acquisition unit 421 may estimate the growth stage by using the number of days elapsed from the planting date, which is in the cultivation information acquired, until the present time as the number of days since planting, and then comparing the number of days since planting with a threshold.

The control device 20 for adjusting the environmental conditions inside the plastic greenhouses 10a-10c also affects the dryness condition. The information acquisition unit 421 may acquire operating information of the control device 20 from the communication device 26, and the learning unit 422 may use this operating information as one item of input data. As a result, a prediction that also takes account of adjustments of the environmental conditions performed by the control device 20 can be made, further improving the prediction accuracy. Examples of the operating information that may be cited include: whether or not a control device 20 is installed, the type of control device 20, an operating condition indicating whether the control device 20 is stopped or operating, a target temperature, and a target humidity, etc.

The learning unit 422 preferably updates the prediction model saved in the memory unit 430 by performing the abovementioned processing periodically or at any time. As a result, predictions based on the most recent trends can be made.

Fig. 5 shows the processing sequence by which the information processing server 40 predicts the risk of diseases and pests.

As shown in fig. 5, in the information processing server 40, the information acquisition unit 421 acquires from the communication device 26 measurement information of the relative humidity measured inside the plastic greenhouses 10a-10c by means of the sensor 22, and stores this measurement information in the memory unit 430 (step S21). The prediction unit 423 generates a feature value representing the dryness condition in the plastic greenhouses 10a-10c from the measurement information of the relative humidity which has been saved, in the same way as when a feature value is generated by the learning unit 422 (step S22).

The prediction unit 423 then inputs the generated feature value to the prediction model and acquires a prediction result of the risk of diseases and pests output from the prediction model (step S23). As described above, the prediction unit 423 may improve the prediction accuracy by using, as the input data for the prediction model, feature values other than the feature value generated from the measurement information of the relative humidity, or various types of information. Examples of the prediction result output from the prediction model that may be cited include the type of disease or pest being predicted, and the predicted rate of infection or rate of disease outbreak in day units, etc.

The prediction unit 423 generates prediction information of diseases and pests on the basis of the prediction result acquired. The prediction information of the risk of diseases and pests includes, for example, information such as the rate of infection or rate of disease outbreak from the diseases and pests, days on which infection or disease outbreak is predicted, and days predicted to be optimum for measures against infection or disease outbreak, such as spraying agrochemicals or controlling environmental conditions. The prediction unit 423 sends the generated prediction information to the user terminal 50 (step S24) .

The user terminal 50 may display the prediction information sent from the information processing server 40.

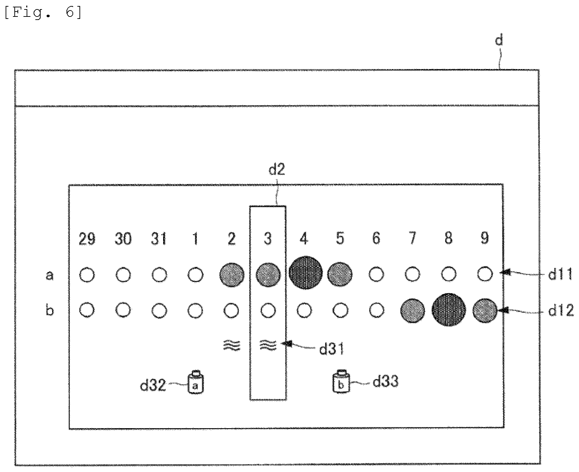

Fig. 6 shows an example of a display screen for the prediction information in the plastic greenhouse 10a.

A risk d11 of infection with a disease a and a risk d12 of infection with a pest b from the 29^{th} day of the previous month until the 9^{th} day of the present month are displayed as one item of prediction information on a display screen d shown in fig. 6. A box mark d2 shows today's date. The risks d11 and d12 are represented by circular marks, with the circles being larger to denote a higher rate of infection, and the circles being darker to denote a higher rate of infection. For example, it is clear from the risk d11 that the rate of infection with the disease a is highest on the 4^{th} day, and it is clear from the risk d12 that the rate of infection with the pest b is highest on the 8^{th} day.

Furthermore, marks d31-d33 indicating days predicted to be optimum for measures against infection with the disease or pest are displayed as one item of prediction information on the display screen d. The mark d31 indicates a day on which it would be effective to control the environmental conditions by means of air conditioning inside the plastic greenhouse 10a. The marks d32 and d33 respectively indicate days on which it would be effective to spray agrochemicals for the disease a and the pest b.

It should be noted that the prediction information shown in fig. 6 is an example, and the prediction information is not limited to this. For example, a graph of the infection rate transitioning in day units or weeks units may equally be provided for each type of disease or pest as the prediction information, in such a way that the user can easily ascertain when and what type of disease or pest has a high risk of occurring.

As described above, the information processing server 40 according to this mode of embodiment generates a feature value representing a dryness condition inside the plastic greenhouses 10a-10c from measurement information of the relative humidity measured inside the plastic greenhouses 10a-10c, and predicts the risk of diseases and pests on the basis of this feature value. As a result, it is possible to accurately predict a risk of diseases and pests that increases under dry conditions. The prediction accuracy is further improved by using the prediction model employing machine learning for the prediction.

The information processing server 40 according to this mode of embodiment also generates a feature value representing a dryness condition from measurement information of the temperature. Furthermore, the information processing server 40 uses, as input data for the prediction model, not only the feature value representing the dryness condition, but also at least one item of information from measurement information of the weather conditions, cultivation information, and operating information of the control device 20, which affect the dryness condition. The greater the amount of input data used for the prediction, the more comprehensive the prediction which can be made, which further improves the prediction accuracy.

A preferred mode of embodiment of the present invention was described above, but the present invention is not limited by this mode of embodiment, and a number of variations and modifications may be made within the scope of the essential point thereof.

For example, the learning unit 422 may be provided in an external device such as another server, rather than in the information processing server 40, and the information processing server 40 may acquire the prediction model generated in the external device and store the prediction model in the memory unit 430.

Furthermore, when the control device 20 for adjusting the environmental conditions inside the plastic greenhouses 10a-10c is provided, prediction information may be sent from the information processing server 40 to the control device 20. The control device 20 may control the environmental conditions inside the plastic greenhouses 10a-10c on the basis of the prediction information.

### Key to Symbols

1... Information provision system, 10a-10c... Plastic greenhouse, 21-23... Sensor, 26... Communication device, 30... Weather server, 40... Information processing server, 421... Information acquisition unit, 422... Learning unit, 423... Prediction unit

## Claims

1. An information processing device (40) comprising:
an information acquisition unit (421) for acquiring measurement information of relative humidity inside a plastic greenhouse; and
a prediction unit (423) for generating a feature value representing a dryness condition inside the plastic greenhouse from the measurement information of the relative humidity, and predicting a risk of diseases and pests inside the plastic greenhouse on the basis of the feature value.

2. The information processing device (40) as claimed in claim 1, wherein the prediction unit (423) uses the feature value generated from the measurement information of the relative humidity for a fixed period in the past in order to further generate a feature value representing a dryness condition inside the plastic greenhouse in the fixed period, and predicts the risk of diseases and pests on the basis of each of the feature values.

3. The information processing device (40) as claimed in claim 1 or 2, wherein the information acquisition unit (421) further acquires measurement information of a temperature inside the plastic greenhouse, and
the prediction unit (423) generates a plurality of feature values representing the dryness condition inside the plastic greenhouse from the measurement information of the relative humidity and the temperature, and predicts the risk of diseases and pests inside the plastic greenhouse on the basis of the plurality of feature values.

4. The information processing device (40) as claimed in any one of claims 1 to 3, wherein the information acquisition unit (421) further acquires at least one item of information from among: measurement information of an environmental condition inside the plastic greenhouse, cultivation information relating to a cultivation condition inside the plastic greenhouse, and measurement information relating to a weather condition outside the plastic greenhouse, and
the prediction unit (423) predicts the risk of diseases and pests on the basis of the feature value representing the dryness condition and at least one of the items of information.

5. The information processing device (40) as claimed in any one of claims 1 to 4, wherein, when one or more feature values including at least the feature value representing the dryness condition is input, the prediction unit (423) predicts the risk of diseases and pests by using a prediction model which outputs the risk of diseases and pests under that dryness condition.

6. The information processing device (40) as claimed in claim 5, comprising a learning unit (422) which uses the one or more feature values as input data, uses the risk of diseases and pests under the dryness condition as teaching data, and generates the prediction model by means of machine learning.

7. The information processing device (40) as claimed in any one of claims 1 to 6, wherein the prediction unit (423) generates prediction information on the basis of a prediction result of the risk of diseases and pests, and sends the prediction information to a user terminal (50).

8. A method for predicting a risk of diseases and pests in a plastic greenhouse, the method comprising:
a step of acquiring measurement information of relative humidity inside the plastic greenhouse; and
a step of generating a feature value representing a dryness condition inside the plastic greenhouse from the measurement information of the relative humidity, and predicting a risk of diseases and pests inside the plastic greenhouse on the basis of the feature value.
